# EUROPEAN PATENT APPLICATION

(11) **EP 4 740 884 A1**
(43) Date of publication of application: **13.05.2026**
(21) Application number: 25214793.9
(22) Date of filing: 11.11.2025
(51) Int. Cl.: A61B 18/14, A61B 18/00

(54) **COATED END EFFECTOR ELECTRODES FOR SENSING AND ABLATION**

(30) Priority: 12.11.2024 US 202418945035
(71) Applicant: Biosense Webster (Israel) Ltd., 2066717 Yokneam (IL)
(72) Inventor: GOVARI, Assaf, 2066717 Yokneam (IL); BEECKLER, Christopher Thomas, Irvine, 92618 (US); KEYES, Joseph, Irvine, 92618 (US)
(74) Representative: Carpmaels & Ransford LLP

(57) **Abstract**

A medical probe can include electrodes for ablation and sensing proximity to tissue and/or monitoring electrical signals within the cardiovascular system to identify aberrant conductive tissue sites that are responsible for an arrhythmia. The electrodes can be oriented on an end effector assembly, such as a basket assembly, such that one side of an electrode is positioned to contact tissue and the opposite side is inhibited from contacting tissue. To enable both efficient delivery of energy as well as high sensitivity for ECG sensing an impedance reducing coating can be positioned on the tissue contacting side and an impedance increasing coating can be applied on the opposite side. The impedance increasing coating of an IRE electrode can be thin enough to allow energy to be delivered through the coating during ablation. An RF electrode an have sufficient mass to carry electrical energy for thermal ablation.

## Description

### FIELD

The present invention relates generally to medical devices, and in particular to medical probes such as catheters configured to sense tissue proximity and delivery ablation therapy.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many contemporaneous catheter-based ablation approaches utilize radiofrequency (RF) electrical energy to heat tissue. Cryoablation is an alternative catheter-based approach to RF ablation that disable electrical signals through tissue with low temperate rather than heat. Irreversible electroporation (IRE) is a recent catheter-based electrical ablation approach to ablate cardiac tissue using nonthermal ablation. To achieve IRE, short pulses of high voltage electrical signals are delivered to tissues; the electrical signals generate an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference.

Regions of cardiac tissue can be mapped by a catheter to identify the abnormal electrical signals. The same or different catheter can be used to perform ablation. Regions of cardiac tissue can be mapped by a catheter to identify the abnormal electrical signals. The same or different catheter may be used to perform ablation. Some catheter ablation procedures, especially those with persistent atrial fibrillation, may be performed using electrophysiology (EP) mapping to target areas of aberrant electrical signals. Such EP mapping may include the use of sensing electrodes configured to monitor electrical signals within the cardiovascular system to pinpoint the location of aberrant conductive tissue sites that are responsible for the arrhythmia. Examples of an EP mapping system are described in U.S. Patent No. 5,738,096, incorporated herein by reference and attached in the Appendix hereto. Examples of EP mapping catheters are described in U.S. Patent No. 9,907,480, U.S. Patent Pub. No. 2018/0036078, and U.S. Patent Pub. No. 2018/0056038, each of which are incorporated herein by reference and attached in the Appendix hereto.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, Calif. Examples of catheters that are configured for use with an IGS system are disclosed in U.S. Patent No. 9,480,416, incorporated herein by reference.

### SUMMARY

A medical probe can include electrodes configured to perform electrical ablation using IRE and/or thermal ablation while also being configured for sensing function such as sensing proximity to tissue and/or monitoring electrical signals within the cardiovascular system to identify aberrant conductive tissue sites that are responsible for an arrhythmia. When configured for thermal ablation, the electrodes have sufficient mass to carry electrical energy and provide thermal conduction and stability for thermal ablation. The electrodes can be oriented on an end effector assembly, such as a basket assembly, such that one side of an electrode is positioned to contact tissue and the opposite side is inhibited from contacting tissue. To enable both efficient delivery of energy as well as high sensitivity for ECG sensing an impedance reducing coating can be positioned on the tissue contacting side and an impedance increasing coating can be applied on the opposite side. When configured for IRE, the impedance increasing coating is configured to provide sufficient electrical resistance to provide specificity for tissue proximity indication measurements while being thin enough to allow electrical energy to be delivered through the impedance increasing coating during IRE.

An example medical probe includes a shaft, a plurality of spines, and a plurality of electrodes. The shaft extends along a longitudinal axis. The plurality of spines extend from a distal end of the shaft and are configured to expand away from the longitudinal axis to form a resilient basket. Each electrode of the plurality of electrodes includes a respective electrically conductive body circumscribing a respective spine of the plurality of spines, an impedance reducing coating on an outer surface of the respective electrically conductive body such that the outer surface faces away from the longitudinal axis, and an impedance increasing coating on an inner surface of the respective electrically conductive body such that the inner surface faces toward the longitudinal axis.

An example method includes providing a medical probe having a shaft extending along a longitudinal axis, a plurality of spines extending from a distal end of the shaft and configured to expand away from the longitudinal axis to form a resilient basket, and a plurality of electrodes each having a respective electrically conductive body circumscribing a respective spine of the plurality of spines; applying an impedance reducing coating to an outer surface of a respectively conductive body of each electrode of the plurality of electrodes such that the outer surface faces away from the longitudinal axis; and applying an impedance increasing coating on an inner surface of the respective electrically conductive body such that the inner surface faces toward the longitudinal axis.

An example system includes a medical probe and a console. The medical probe includes a shaft, one or more spines extending from a distal end of the shaft, and a plurality of electrodes each having a respective electrically conductive body circumscribing a respective spine of the one or more spines. Each of the plurality of electrodes has an impedance reducing coating on a first surface of the respective electrically conductive body and an impedance increasing coating on a second surface of the respective electrically conductive body. The console includes at least one processor and non-transitory computer-readable medium in communication with the at least one processor. The non-transitory computer-readable medium includes instructions thereon, that when executed by the processor, cause the console to sense, based in part on an impedance measurement between one or more electrode pairs of the plurality of electrodes, contact of at least a portion of the plurality of electrodes with tissue; and provide electrical energy to at least a portion of the plurality of electrodes to ablate tissue.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements. The figures depict one or more implementations of the inventive devices, by way of example only, not by way of limitation.
Figure 1 is an illustration of an example catheter-based electrophysiology mapping and ablation system according to aspects of the present invention.
Figure 2 is an illustration of a distal portion of a catheter according to aspects of the present invention.
Figures 3A and 3B are illustrations of opposite sides of an electrode according to aspects of the present invention.
Figure 4 is a flow diagram of a method 100 for configuring coated electrodes for electrocardiogram sensing.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives, and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away from the operator or physician.

Alternative apparatus and system features and alternative method steps are presented in example embodiments herein. Each given example embodiment presented herein can be modified to include a feature and/or method step presented with a different example embodiment herein where such feature and/or step is compatible with the given example as understood by a person skilled in the pertinent art as well as where explicitly stated herein. Such modifications and variations are intended to be included within the scope of the claims.

Electrodes positioned on an end effector of a catheter which are configured for energy delivery and ECG sensing are typically selected to have a low impedance to enable both efficient delivery of energy as well as high sensitivity for ECG sensing. Electrodes with high energy delivery, particularly for delivery of RF electrical signals for thermal ablation, require enough mass to carry the electrical and thermal energy. For this purpose, cylindrically shaped electrodes may be used because cylindrically shaped electrodes have greater mass than alternative electrode shapes such as surface mount electrodes and/or flex circuit electrodes. Cylindrically shaped electrodes, have straight, parallel sides and a circular, oval, or rounded rectangular cross-section with a central opening. The cylindrically shape electrodes can be threaded onto spines of an end effector at a distal end of a medical probe. Cylindrically shaped electrodes are an example of an electrode shape which includes an electrically conductive body which circumscribes a spine of an end effector; however, examples herein may be modified to include electrodes having alternative shapes as understood by a person skilled in the pertinent art.

Local tissue proximity indicator (TPI) detection is based on sensing impedance between electrodes. The impedance detected significantly increases when the electric signal between the electrodes passes through or near a tissue wall as compared to when the electrical signal entirely passes through the blood pool. When using cylindrically shaped electrodes, the electric signal is distributed across the entire electrode, front and back. However, the backside of the electrode will always be in the blood pool. Because of the low impedance contact of the backside of the electrode to blood, the overall detected change in impedance when the frontside of the electrode touches tissue can be small. Assessment of tissue contact can be determined based sensing impedance between electrodes by various methods, for example as disclosed in U.S. Patent No. 11,523,750 incorporated by reference herein and attached in the Appendix hereto.

Embodiments presented herein include an impedance reducing coating on the frontside of electrodes and an impedance increasing coating on the backside of electrodes. As a result, the impedance on the backside of the electrode through blood is increased and the impedance on the frontside of the contact changes more dramatically when in contact with blood vs. tissue. As a result, the overall change in impedance between electrodes when an electrode comes into contact with tissue is greater compared to a similarly configured electrode without the backside and frontside coatings. Therefore, the electrode coatings, in at least some applications, can make TPI impedance measurements more sensitive. Some embodiments present catheters configured for IRE. In such embodiments, the impedance increasing coating is configured to provide sufficient electrical resistance to provide specificity for tissue proximity indication measurements while being thin enough to allow electrical energy to be delivered through the impedance increasing coating during IRE. An example of a suitable impedance reducing coating is Amplicoat^{®}, available from Heraeus Medevio. Amplicoat^{®} is a conductive polymer technology that is biocompatible. The Amplicoat^{®} coating may be applied with electrodeposition that grows a coated layer with current being passed across the surface. Alternative conductive coatings, such as TiOx and IrOx, can be utilized as understood by a person skilled in the pertinent art. An example of a suitable impedance increasing coating is silicon nitride (Si₃N₄). The silicon nitride coating may be applied with aerosol deposition. In examples illustrated herein, the cassette, shape of the electrode, facilitates the separation between the two coatings in an easy and efficient manner.

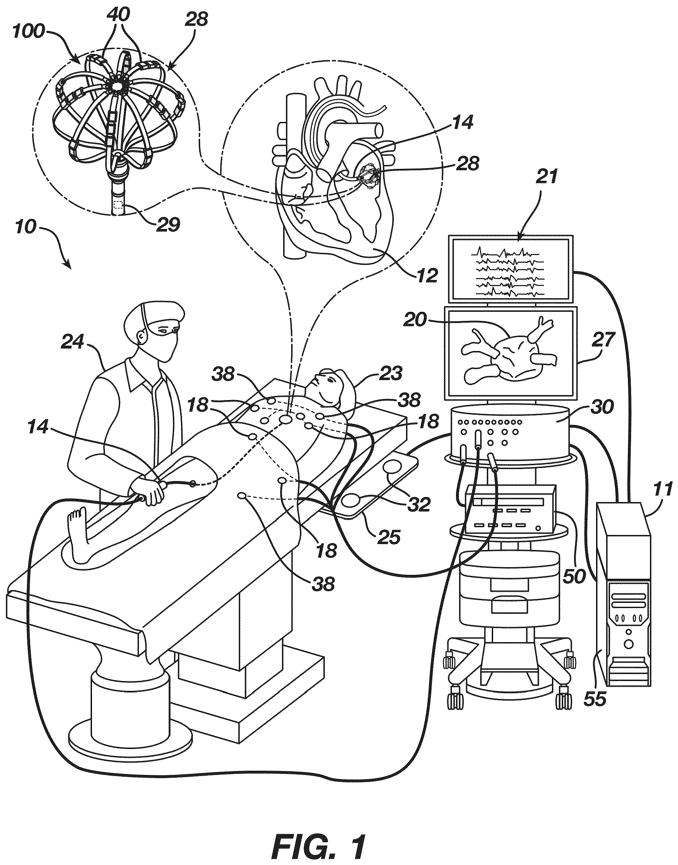

Figure 1 is an illustration showing an example catheter-based electrophysiology mapping and ablation system 10. The system 10 includes multiple catheters, which are percutaneously inserted by a physician 24 through the patient's vascular system into a chamber or vascular structure of a heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for IRE and/or RF ablation is illustrated herein. In some embodiments, the catheter 14 is also configured for IEGM sensing. When configured for IRE, having accurate tissue proximity indication is important to ensure that the catheter is in proper contact with tissue to perform an ablation. When configured for RF ablation, it is important for the electrode to have sufficient thermal mass to perform thermal ablation. The physician 24 brings a distal tip 28 of the catheter 14 into contact with the heart wall for sensing a target site in the heart 12. For ablation, the physician 24 would similarly bring a distal end of an ablation catheter to a target site for ablating.

The illustrated catheter 14 is an exemplary catheter that includes one and preferably multiple electrodes 40 optionally distributed over a plurality of spines 22 at distal tip 28. As illustrated, the spines 22 are shaped to form a resilient basket, referred to herein as a basket assembly 100. The electrodes 40 are configured for providing RF ablation energy to tissue and function as TPI sensors. Some, or preferably all, of the electrodes 40 include an impedance reducing coating 42 on an outer surface (frontside) and an impedance increasing coating 44 on an inner surface (backside) to increase sensitivity of TPI measurements.

Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation. A magnetic based position sensor 29 may be operated together with a location pad 25 including a plurality of magnetic coils 32 configured to generate magnetic fields in a predefined working volume. Real time position of a distal tip 28 of the catheter 14 may be tracked based on magnetic fields generated with a location pad 25 and sensed by a magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091 incorporated by reference herein.

The system 10 includes one or more electrode patches 38 positioned for skin contact on the patient 23 to establish a location reference for location pad 25. The distal tip 28 of the catheter may include as impedance-based tracking of electrodes 40 or additional electrodes (not illustrated). For impedance-based tracking, electrical current is directed toward electrodes at a distal end of a catheter and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182 incorporated by reference herein.

A recorder 11 displays electrograms 21 captured with body surface ECG electrodes 18 and intracardiac electrograms (IEGM) captured with electrodes 40 of the catheter 14. The recorder 11 may include pacing capability for pacing the heart rhythm and/or may be electrically connected to a standalone pacer.

The system 10 can include an ablation energy generator 50 that is adapted to conduct ablative energy to the electrodes 40. Energy produced by the ablation energy generator 50 may include, but is not limited to, radio frequency (RF) energy or pulsed-field ablation (PFA) energy, including monopolar or bipolar high-voltage DC pulses as may be used to effect irreversible electroporation (IRE), or combinations thereof.

A patient interface unit (PIU) 30 is an interface configured to establish electrical communication between catheters, electrophysiological equipment, power supply and a workstation 55 for controlling operation of system 10. Electrophysiological equipment of the system 10 may include for example, multiple catheters (including catheter 14), a location pad 25, body surface ECG electrodes 18, electrode patches 38, an ablation energy generator 50, and a recorder 11. Optionally and preferably, the PIU 30 includes processing capability for implementing real-time computations of location of the catheters, sensing tissue proximity of electrodes 40, controlling ablation energy to electrodes 40, and for performing ECG calculations.

The workstation 55 includes memory, processor unit with memory or storage with appropriate operating software loaded therein, and user interface capability. The workstation 55 can be configured to provide multiple functions, optionally including (1) modeling the endocardial anatomy in three-dimensions (3D) and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on the display device 27 sites of interest such as places where ablation energy has been applied. One commercial product embodying elements of the system 10 is available as the CARTO^{™} 3 System, available from Biosense Webster, Inc., California.

The PIU 30 and/or workstation 55 includes a console that is housed in the PIU 30 and/or workstation 55 or distributed between the PIU 30 and workstation 55. The console is configured to control ablation output and measure tissue proximity via electrodes 40. The console includes at least one processor and non-transitory computer-readable medium in communication with the at least one processor. The non-transitory computer-readable medium includes instructions thereon, that when executed by the processer cause the console to sense contact of at least a portion of the plurality of electrodes with tissue based in part on an impedance measurement between one or more electrode pairs of the plurality of electrodes and provide electrical energy to at least a portion of the plurality of electrodes to ablate tissue.

The impedance measurement between the electrode pair(s) can be based at least in part on an impedance of the impedance reducing coating 42 and an impedance of the impedance increasing coating 44 of the electrodes 40. The console can be configured to sense contact of at least a portion of the electrodes 40 with tissue based in part on the impedance measurement between the one or more electrode pairs and assuming that the impedance increasing coating of each electrode of the pairs is in contact with blood. Optionally, the console can also be configured to generate electrocardiograms based at least in part on electrical signals received from at least a portion of the electrodes 40.

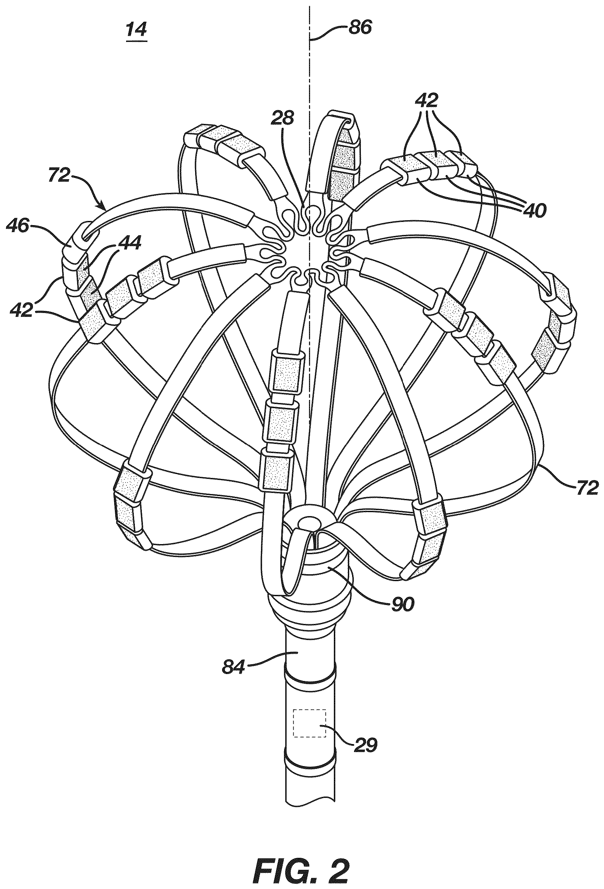

Figure 2 is an illustration of the distal end 28 of the catheter 14 showing a distal portion of the shaft 84 and an end effector having spines 72 and electrodes 40. The shaft extends along a longitudinal axis 86. The spines 72 extend from a distal end 90 of the shaft 84 and are configured to expand away from the longitudinal axis 84 to form a resilient basket. The electrodes each have a respective electrically conductive body, an impedance reducing coating 42, and an impedance increasing coating 44. The electrically conductive body circumscribes a respective spine 72. The electrically conductive body is oriented such that an outer surface of the electrically conductive body faces away from the longitudinal axis an inner surface of the electrically conductive body faces toward the longitudinal axis. As such, the inner surface is inhibited from contacting tissue and will therefore be in contact with blood (or other bodily fluid) when the end effector is deployed in a patient. The outer surface is positioned so that it is able to contact tissue. The electrodes 40 include an impedance reducing coating 42 on an outer surface of the respective electrically conductive body and an impedance increasing coating 44 on the inner surface of the respective electrically conductive body.

The electrodes 40 are each configured to sense tissue contact and provide ablation energy. In some embodiments, the impedance reducing coating includes a conductive polymer. In some embodiments, the impedance increasing coating includes a ceramic. In some embodiments, the impedance increasing coating has a thermal conductivity of approximately 7 W/mK to approximately 30 W/mK; or approximately 7 W/mK to approximately 28 W/mK; or approximately 30 W/mK. In some embodiments, the impedance increasing coating includes silicon nitride. In some embodiments, the electrodes 40 are configured for electrocardiogram sensing.

As understood by a person skilled in the pertinent art, the end effector can have various alternative configurations, including alternative basket assembly configurations as well as ray or spade configurations. In such configurations, electrodes 40 can be positioned and otherwise configured with an impedance reducing coating 42 on a surface that is able to contact tissue and an impedance increasing coating 44 on a surface that is inhibited from contacting tissue when the end effector is deployed in a patient as understood by a person skilled in the pertinent art.

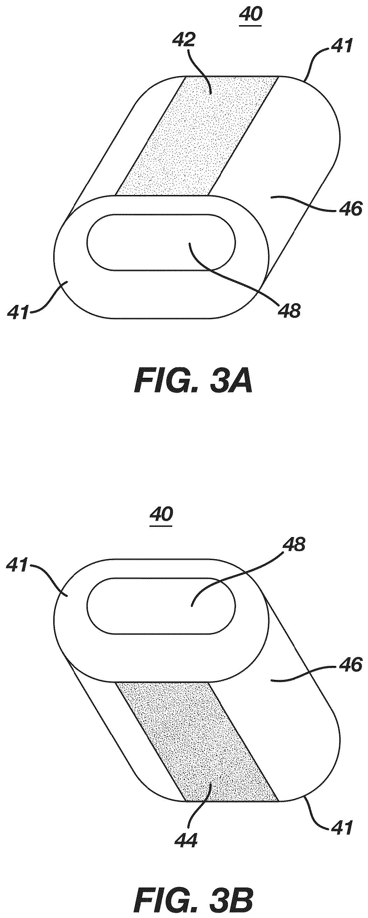

Figures 3A and 3B are illustrations of opposite sides of an electrode 40. The electrode 40 has an electrically conductive body 46 with a cylindrical shape with an opening 48 therethrough. The size of the electrically conductive body 46 is configured to provide sufficient thermal mass so that the electrode 40 is able to deliver RF energy to tissue without incurring thermal damage to the end effector. The opening 48 is sized to receive a spine 72 so that the electrode 40 can be threaded onto a spine 72 during manufacturing. As illustrated, the cross-section of the electrically conductive body 46 has a rounded rectangle or stadium shape with linear sides corresponding to the outer surface and the inner surface and rounded sides extending between the outer surface and the inner surface. The shape of the electrically conductive body 46 facilitates the application of the impedance reducing coating 42 and the impedance increasing coating 44 on opposite sides of the conductive body 46 without overlapping. The electrically conductive body 46 can have alternative cross-sectional shapes as understood by a person skilled in the pertinent art (e.g., circular, rectangular, oval, triangular, etc.). As illustrated, the coatings 42, 44 do not significantly affect the overall shape of the electrode 40.

As illustrated, the opening 48 is also a rounded rectangle or stadium shape. As illustrated, the opening 48 is centered left to right and offset from the center toward the outer surface. The opening 48 can be offset to provide desired thermal conduction to tissue and thermal dissipation to blood.

Figure 3A shows a frontside or outer surface of the electrode 40 showing the impedance reducing coating 42 on the frontside or outer surface of the electrically conductive body 46. As illustrated, the impedance reducing coating 42 is confined to a flat portion of the outer surface. The outer surface is shaped so that, during a treatment, when in contact with tissue, the entirety of the flat surface is in contact with tissue while curved edges of the outer surface are partially in contact with tissue. An advantage of this configuration is that when the outer surface is in contact with tissue, the entirety of the impedance reducing coating 42 is in contact with tissue with no portion in contact with blood. A low resistance path through blood reduces TPI sensitivity. By positioning the impedance reducing coating 42 so that all of the coating 42 is in contact with tissue, the coating is not providing a low resistance path through blood when the electrode 40 is in contact with tissue. Alternatively, the impedance reducing coating 42 can be applied over a portion of a curved surface of the outer surface.

Figure 3B shows a backside or inner surface of the electrode 40 showing the impedance increasing coating 44 on the backside or inner surface of the electrically conductive body 46. As illustrated, the impedance increasing coating 44 is confined to a flat portion of the inner surface. The inner surface can be shaped to facilitate movement of the end effector (e.g., expansion and collapse of a basket assembly). The impedance increasing coating 44 can be confined to regions that are not intended, expected, and/or configured to come into contact with tissue when the end effector is deployed in a patient. Alternative, the impedance increasing coating 44 can extend around the surface of the electrically conductive body 46 and end adjacent to the impedance reducing coating 42.

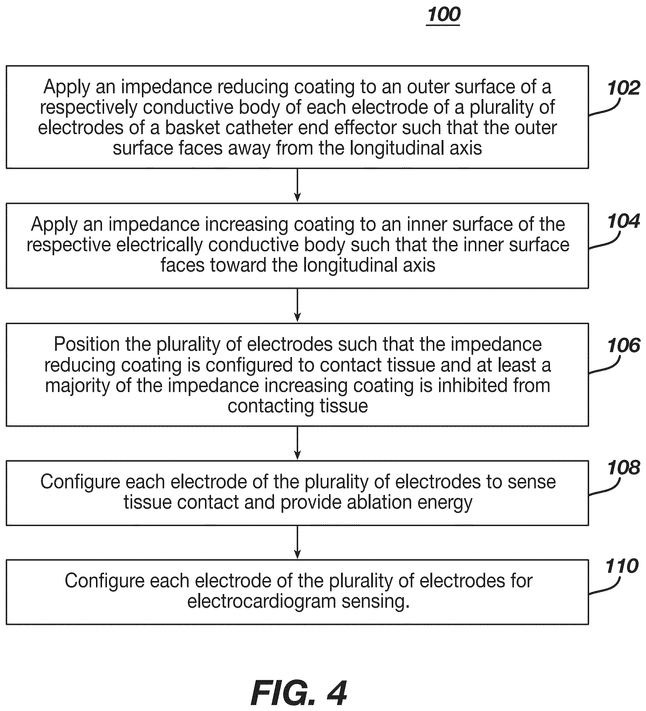

Figure 4 is a flow diagram of a method 100 for configuring coated electrodes for electrocardiogram sensing. The resulting electrodes can be configured similar to electrodes 40, alternative thereto, or variation thereof as understood by a person skilled in the pertinent art according to the disclosure herein.

At block 102, an impedance reducing coating can be applied to an outer surface of a respectively conductive body of each electrode of a plurality of electrodes of a basket catheter end effector such that the outer surface faces away from the longitudinal axis. In some embodiments, the impedance reducing coating is applied by electrodeposition. In some embodiments, the impedance reducing coating includes a conductive polymer. The impedance reducing coating can include materials and be applied as disclosed elsewhere herein, alternatives thereto, or variations thereof as understood by a person skilled in the pertinent art according to the disclosure herein. The outer surface can be shaped, positioned, and otherwise configured as disclosed elsewhere herein, alternatives thereto, or variations thereof as understood by a person skilled in the pertinent art according to the disclosure herein.

At block 104, an impedance increasing coating can be applied to an inner surface of the respective electrically conductive body such that the inner surface faces toward the longitudinal axis. In some embodiments, the impedance increasing coating includes a ceramic. In some embodiments, the impedance increasing coating includes a thermal conductivity of approximately 30 W/mK. In some embodiments, the impedance increasing coating includes silicon nitride. The impedance increasing coating can include materials and be applied as disclosed elsewhere herein, alternatives thereto, or variations thereof as understood by a person skilled in the pertinent art according to the disclosure herein. The inner surface can be shaped, positioned, and otherwise configured as disclosed elsewhere herein, alternatives thereto, or variations thereof as understood by a person skilled in the pertinent art according to the disclosure herein.

At block 106, the plurality of electrodes can be positioned such that the impedance reducing coating is configured to contact tissue and at least a majority of the impedance increasing coating is inhibited from contacting tissue. The coatings can be positioned on an electrically conductive body of an electrode as disclosed elsewhere herein, alternatives thereto, or variations thereof as understood by a person skilled in the pertinent art according to the disclosure herein.

At block 108, each electrode of the plurality of electrodes can be configured to sense tissue contact and provide ablation energy. The electrodes can be configured to sense tissue contact using TPI measurement techniques disclosed elsewhere herein, alternatives thereto, or variations thereof as understood by a person skilled in the pertinent art according to the disclosure herein. The coatings can be positioned on the electrodes to increase sensitivity of TPI measurements. The electrodes can be shaped, sized, and otherwise configured to provide ablation energy as disclosed elsewhere herein, alternatives thereto, or variations thereof as understood by a person skilled in the pertinent art according to the disclosure herein.

At block 110, each electrode of the plurality of electrodes can be configured for electrocardiogram sensing. The electrodes can be configured to sense electrical signals through cardiac tissue for electrocardiogram measurement using techniques disclosed elsewhere herein, alternatives thereto, or variations thereof as understood by a person skilled in the pertinent art according to the disclosure herein.

The following clauses list non-limiting embodiments of the disclosure:
Clause 1. A medical probe comprising: a shaft (84) extending along a longitudinal axis (86); a plurality of spines (72) extending from a distal end (90) of the shaft (84) and configured to expand away from the longitudinal axis to form a resilient basket; and a plurality of electrodes (40), each electrode of the plurality of electrodes comprising: a respective electrically conductive body circumscribing a respective spine of the plurality of spines, an impedance reducing coating (42) on an outer surface of the respective electrically conductive body such that the outer surface faces away from the longitudinal axis, and an impedance increasing coating (44) on an inner surface of the respective electrically conductive body such that the inner surface faces toward the longitudinal axis.
Clause 2. The medical probe of clause 1, wherein each electrode of the plurality of electrodes is configured to sense tissue contact and provide ablation energy.
Clause 3. The medical probe of clause 1 or 2, wherein the impedance reducing coating comprises a conductive polymer.
Clause 4. The medical probe of any one of clauses 1-3, wherein the impedance increasing coating comprises a ceramic.
Clause 5. The medical probe of any one of clauses 1-4, wherein the impedance increasing coating comprises a thermal conductivity of approximately 30 W/mK.
Clause 6. The medical probe of any one of clauses 1-5, wherein the impedance increasing coating comprises Si₃N₄.
Clause 7. The medical probe of any one of clauses 1-6, wherein each electrode of the plurality of electrodes is configured for electrocardiogram sensing.
Clause 8. The medical probe of any one of clauses 1-7, wherein the impedance reducing coating is thinner than the impedance increasing coating.
Clause 9. The medical probe of any one of clauses 1-8, wherein the plurality of electrodes are configured to provide irreversible electroporation to tissue.
Clause 10. The medical probe of any one of clauses 1-9, wherein the plurality of electrodes are configured to provide radio frequency ablation to tissue.
Clause 11. The medical probe of any one of clauses 1-10, wherein the impedance reducing coating comprises a lower impedance than the impedance increasing coating.
Clause 12. The medical probe of clause 11, wherein difference in impedance between the impedance reducing coating and the impedance increasing coating is smaller when applying irreversible electroporation than difference in impedance between the impedance reducing coating and the impedance increasing coating during electrocardiogram sensing.
Clause 13. A method, comprising: providing a medical probe comprising a shaft extending along a longitudinal axis, a plurality of spines extending from a distal end of the shaft and configured to expand away from the longitudinal axis to form a resilient basket, and a plurality of electrodes each comprising a respective electrically conductive body circumscribing a respective spine of the plurality of spines; applying an impedance reducing coating to an outer surface of a respectively conductive body of each electrode of the plurality of electrodes such that the outer surface faces away from the longitudinal axis; and applying an impedance increasing coating on an inner surface of the respective electrically conductive body such that the inner surface faces toward the longitudinal axis.
Clause 14. The method of clause 13, wherein applying the impedance reducing coating comprises electrodepositing the impedance reducing coating.
Clause 15. The method of clause 13 or 14, wherein applying the impedance increasing coating comprises aerosol depositing the impedance increasing coating.
Clause 16. The method of any one of clauses 13-15, wherein the impedance reducing coating comprises a conductive polymer.
Clause 17. The method of any one of clauses 13-16, wherein the impedance increasing coating comprises a ceramic.
Clause 18. The method of any one of clauses 13-17, wherein the impedance increasing coating comprises a thermal conductivity of approximately 30 W/mK.
Clause 19. The method of any one of clauses 13-18, wherein the impedance increasing coating comprises Si₃N₄.
Clause 20. The method of any one of clauses 13-19, wherein applying the impedance reducing coating comprises configuring the impedance reducing coating to contact tissue, and wherein applying the impedance increasing coating comprises positioning the impedance increasing coating such that at least a majority of the impedance increasing coating is inhibited from contacting tissue.
Clause 21. The method of any one of clauses 13-20, further comprising: configuring each electrode of the plurality of electrodes to sense tissue contact and provide ablation energy.
Clause 22. The method of any one of clauses 13-21, further comprising: configuring each electrode of the plurality of electrodes to sense electrocardiograms.
Clause 22. The method of any one of clauses 13-21, wherein the impedance reducing coating is thinner than the impedance increasing coating.
Clause 23. The method of any one of clauses 13-22 comprising configuring the plurality of electrodes to provide irreversible electroporation to tissue.
Clause 24. The method of any one of clause 13-23 comprising configuring the plurality of electrodes to provide radio frequency ablation to tissue.
Clause 25. The method of any one of clauses 13-24, wherein the impedance reducing coating comprises a lower impedance than the impedance increasing coating.
Clause 26. The method of any one of clauses 13-25 comprising configuring the plurality of electrodes such that difference in impedance between the impedance reducing coating and the impedance increasing coating is smaller when applying irreversible electroporation than difference in impedance between the impedance reducing coating and the impedance increasing coating during electrocardiogram sensing.
Clause 27. A system comprising: a medical probe comprising a shaft, one or more spines extending from a distal end of the shaft, and a plurality of electrodes each comprising a respective electrically conductive body circumscribing a respective spine of the one or more spines, each of the plurality of electrodes comprising an impedance reducing coating on a first surface of the respective electrically conductive body and an impedance increasing coating on a second surface of the respective electrically conductive body; and a console comprising at least one processor and non-transitory computer-readable medium in communication with the at least one processor and comprising instructions thereon, that when executed by the processor, cause the console to: sense, based in part on an impedance measurement between one or more electrode pairs of the plurality of electrodes, contact of at least a portion of the plurality of electrodes with tissue, and provide electrical energy to at least a portion of the plurality of electrodes to ablate tissue.
Clause 28. The system of clause 27, wherein the impedance measurement between the one or more electrode pairs is based at least in part on an impedance of the impedance reducing coating and an impedance of the impedance increasing coating of each electrode of the one or more electrode pairs.
Clause 29. The system of clause 27 or 28, wherein the non-transitory computer-readable medium comprises instructions thereon, that when executed by the processor, cause the console to: sense, based in part on the impedance measurement between the one or more electrode pairs and assuming that the impedance increasing coating of each electrode of the one or more electrode pairs is in contact with blood, contact of at least a portion of the plurality of electrodes with tissue.
Clause 30. The system of any one of clauses 27-29, wherein the electrical energy comprises radiofrequency ablation energy.
Clause 31. The system of any one of clauses 27-30, wherein the impedance reducing coating comprises a conductive polymer.
Clause 32. The system of any one of clauses 27-31, wherein the impedance increasing coating comprises a ceramic.
Clause 33. The system of any one of clauses 27-32, wherein the impedance increasing coating comprises a thermal conductivity of approximately 30 W/mK.
Clause 34. The system of any one of clauses 27-33, wherein the impedance reducing coating is thinner than the impedance increasing coating.
Clause 35. The system of any one of clauses 27-34, wherein the console is configured to provide electrical pulses to the plurality of electrodes to provide irreversible electroporation to tissue.
Clause 36. The system of any one of clauses 27-35, wherein the console is configured to provide an electrical waveform to the plurality of electrodes to provide radio frequency ablation to tissue.
Clause 37. The system of any one of clauses 27-36, wherein the impedance reducing coating comprises a lower impedance than the impedance increasing coating.
Clause 38. The system of any one of clauses 27-37, wherein difference in impedance between the impedance reducing coating and the impedance increasing coating is smaller when applying irreversible electroporation than difference in impedance between the impedance reducing coating and the impedance increasing coating during electrocardiogram sensing.

Having shown and described exemplary embodiments of the subject matter contained herein, further adaptations of the methods and systems described herein may be accomplished by appropriate modifications without departing from the scope of the claims. For instance, alternative materials can be used for electrode conductive body, impedance reducing coating, impedance increasing coating. The medical probe can have alternative configurations for alternative non-invasive *in vivo* tissue ablation treatments. Spines of the end effector can have alternative configurations such as star, ray, spade, etc. In addition, where methods and steps described above indicate certain events occurring in certain order, it is intended that certain steps do not have to be performed in the order described, but in any order, as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A medical probe comprising:
a shaft (84) extending along a longitudinal axis (86);
a plurality of spines (72) extending from a distal end (90) of the shaft (84) and configured to expand away from the longitudinal axis to form a resilient basket; and
a plurality of electrodes (40), each electrode of the plurality of electrodes comprising:
a respective electrically conductive body circumscribing a respective spine of the plurality of spines,
an impedance reducing coating (42) on an outer surface of the respective electrically conductive body such that the outer surface faces away from the longitudinal axis, and
an impedance increasing coating (44) on an inner surface of the respective electrically conductive body such that the inner surface faces toward the longitudinal axis.

2. The medical probe of claim 1, wherein each electrode of the plurality of electrodes is configured to sense tissue contact and provide ablation energy.

3. The medical probe of claim 1, wherein the impedance reducing coating comprises a conductive polymer and/or the impedance increasing coating comprises a ceramic.

4. The medical probe of any preceding claim, wherein the impedance increasing coating comprises a thermal conductivity of approximately 7 W/mK to approximately 30 W/mK.

5. The medical probe of any preceding claim, wherein each electrode of the plurality of electrodes is configured for electrocardiogram sensing.

6. A method, comprising:
providing a medical probe comprising a shaft extending along a longitudinal axis, a plurality of spines extending from a distal end of the shaft and configured to expand away from the longitudinal axis to form a resilient basket, and a plurality of electrodes each comprising a respective electrically conductive body circumscribing a respective spine of the plurality of spines;
applying an impedance reducing coating to an outer surface of a respectively conductive body of each electrode of the plurality of electrodes such that the outer surface faces away from the longitudinal axis; and
applying an impedance increasing coating on an inner surface of the respective electrically conductive body such that the inner surface faces toward the longitudinal axis.

7. The method of claim 6, wherein applying the impedance reducing coating comprises electrodepositing the impedance reducing coating and/or aerosol depositing the impedance increasing coating.

8. The method of any one of either of claims 6 or 7, wherein the impedance reducing coating comprises a conductive polymer and/or the impedance increasing coating comprises a ceramic.

9. A system comprising:
a medical probe comprising a shaft, one or more spines extending from a distal end of the shaft, and a plurality of electrodes each comprising a respective electrically conductive body circumscribing a respective spine of the one or more spines, each of the plurality of electrodes comprising an impedance reducing coating on a first surface of the respective electrically conductive body and an impedance increasing coating on a second surface of the respective electrically conductive body; and
a console comprising at least one processor and non-transitory computer-readable medium in communication with the at least one processor and comprising instructions thereon, that when executed by the processor, cause the console to:
sense, based in part on an impedance measurement between one or more electrode pairs of the plurality of electrodes, contact of at least a portion of the plurality of electrodes with tissue, and
provide electrical energy to at least a portion of the plurality of electrodes to ablate tissue.

10. The system of claim 9, wherein the impedance measurement between the one or more electrode pairs is based at least in part on an impedance of the impedance reducing coating and an impedance of the impedance increasing coating of each electrode of the one or more electrode pairs.

11. The system of either of claims 9 and 10, wherein the non-transitory computer-readable medium comprises instructions thereon, that when executed by the processor, cause the console to:
sense, based in part on the impedance measurement between the one or more electrode pairs and assuming that the impedance increasing coating of each electrode of the one or more electrode pairs is in contact with blood, contact of at least a portion of the plurality of electrodes with tissue.

12. The system of any of claims 9 to 11, wherein the electrical energy comprises radiofrequency ablation energy.

13. The system of any of claims 9 to 12, wherein the impedance reducing coating comprises a conductive polymer and/or the impedance increasing coating comprises a ceramic.

14. The system of any of claims 9 to 13, wherein the impedance increasing coating comprises a thermal conductivity of approximately 30 W/mK.

15. The medical probe of any of claims 1 to 4 or the system of any of claims 9 to 14, wherein the impedance increasing coating comprises Si₃N₄.
